# EUROPÄISCHE PATENTANMELDUNG

(11) **EP 4 793 966 A1**
(43) Veröffentlichungstag der Anmeldung: **19.08.2026**
(21) Anmeldenummer: 25158374.6
(22) Anmeldetag: 17.02.2025
(51) Int. Cl.: G16H 40/40, G16H 40/63, G06Q 10/20

(54) **VERFAHREN ZUR GENERIERUNG UND AUSGABE EINER ARBEITSANWEISUNG UND MEDIZINISCHES SYSTEM**

(71) Anmelder: B. Braun Melsungen AG, 34212 Melsungen (DE)
(72) Erfinder: KULLMANN, Markus, 36211 Alheim (DE)
(74) Vertreter: Winter, Brandl - Partnerschaft mbB

(57) **Zusammenfassung**

Die vorliegende Offenbarung betrifft ein Verfahren zur Generierung und Ausgabe einer Arbeitsanweisung mit den folgenden Schritten: Erfassen von geräte-spezifischen Informationen eines medizinischen Geräts (1) und/oder eines Bauteils des medizinischen Geräts (1); Auslesen oder Abrufen von reparatur-relevanten Informationen basierend auf den erfassten geräte-spezifischen Informationen, insbesondere aus einer zentralen Datenverarbeitungseinheit (2); Ableiten der Arbeitsanweisung aus den ausgelesenen reparatur-relevanten Informationen; Ausgeben der abgeleiteten Arbeitsanweisung an einen Benutzer, insbesondere über eine Ausgabeeinheit (8); und Überprüfen, insbesondere durch das medizinische Gerät (1), ob die Maßnahme abgeschlossen ist.

## Beschreibung

Die vorliegende Erfindung betrifft ein Verfahren zur Generierung und Ausgabe einer Arbeitsanweisung und ein System mit einem medizinischen Gerät und einer zentralen Datenverarbeitungseinheit.

### Hintergrund der Offenbarung

Im Servicefall oder bei der Reparatur von medizinischen Geräten, wie medizinischen Pumpen oder Dialysegeräten, gehen die Servicetechniker nach einem Handbuch vor. In ihrem Produktlebenszyklus durchlaufen die medizinischen Geräte Änderungen, wie beispielsweise Designänderungen, Komponentenänderungen oder Änderungen und Updates in der Software bzw. im Betriebssystem. Diese Änderungen können auch Auswirkungen auf die Reparatur oder den Service der Geräte haben und müssen deshalb im Handbuch/ Serviceheft reflektiert sein. Beispielsweise können zwei unterschiedliche Bauteile nur miteinander, das heißt in Kombination kompatibel sein. D.h. wenn eines dieser Bauteile ausgetauscht wird, muss das andere Bauteil zwingend mit ausgetauscht werden. Genauso können bestimmte neu eingebaute Bauteile eine jeweilige Softwareversion benötigen, um zu funktionieren. Somit werden durch die Änderungen im Produktlebenszyklus Abhängigkeiten zwischen verschiedenen Bauteilen, Varianten von Bauteilen und/oder Softwarevarianten geschaffen. Diese Abhängigkeiten haben Auswirkungen auf Instandhaltung und Reparatur der Geräte. Zum jetzigen Stand sind diese Abhängigkeiten meist im Handbuch in Abhängigkeit von der Seriennummer des jeweiligen Geräts oder Bauteils beschrieben. Bei steigender Produkt- und Variantenvielfalt nimmt somit die Übersichtlichkeit der Handbücher deutlich ab. Es kann für Servicetechniker schwierig und zeitaufwändig werden, die richtige Reparatur- bzw. Wartungsanweisung für das richtige Modell bzw. für die richtigen Baugruppen zu finden. Das ist eine Fehlerquelle und nimmt auch mehr Zeit in Anspruch und führt damit zu höheren Personal- und Reparaturkosten.

Werden neue Bauteile in ein Gerät eingebaut, können diese neuen Bauteile wieder neue Abhängigkeiten für die nächste Reparatur oder Wartung schaffen. Deshalb werden die Produktnummern dieser neuen Bauteile in einem Gerätebuch, das beispielsweise ein gerätespezifisches Handbuch oder Serviceheft sein kann, üblicherweise händisch dokumentiert. Auch das ist eine potentielle Fehlerquelle.

Ferner gibt es im Bereich der Medizintechnik zum Teil strenge Dokumentationsvorschriften, was die Wartung bzw. Instandhaltung von medizinischen Geräten betrifft. Derzeitig müssen durchgeführte Wartungs- oder Instandhaltungsarbeiten von den Servicetechnikern bzw. Benutzern händisch dokumentiert werden. Wenn diese Dokumentation vergessen wird oder fehlerhaft durchgeführt wird, kann dies eine Fehlerquelle bei weiteren Arbeiten am Gerät darstellen.

### Kurzbeschreibung der Offenbarung

Die Aufgabe der vorliegenden Offenbarung liegt also darin, die Nachteile des Standes der Technik zu überwinden oder zumindest zu mindern und insbesondere darin, ein (automatisiertes) Verfahren bereitzustellen, durch das eine korrekte, auf das vorliegende Produkt angepasste und möglichst einfach zu befolgende Reparaturanweisung bereitgestellt wird. Vorzugsweise soll auch eine (automatisierte) Dokumentation einer durchgeführten Instandhaltung bereitgestellt werden.

Diese Aufgabe wird offenbarungsgemäß durch ein Verfahren nach Anspruch 1 gelöst. Ferner wird die Aufgabe der vorliegenden Offenbarung durch ein System nach Anspruch 9 gelöst. Außerdem wird die Aufgabe durch ein computerlesbares Speichermedium nach Anspruch 12 und ein Computerprogramm nach Anspruch 13 gelöst. Vorteilhafte Weiterentwicklungen der Offenbarung sind Gegenstand der beigefügten Unteransprüche.

Die vorliegende Offenbarung betrifft ein Verfahren zur (automatischen) Generierung einer (geräte- oder variantenspezifischen) Arbeitsanweisung/ Reparaturanleitung/ Serviceanleitung/ Instandhaltungsanleitung mit den folgenden Schritten: Geräte-spezifische Informationen eines medizinischen Geräts und/oder von einzelnen Bauteilen/ Baugruppen werden erfasst. Reparatur-relevante Informationen basierend auf den erfassten geräte-spezifischen Informationen werden ausgelesen/ abgerufen. Die reparatur-relevanten Informationen können dabei insbesondere aus einer zentralen Datenverarbeitungseinheit ausgelesen/ abgerufen werden. Ferner wird die Arbeitsanweisung aus den ausgelesenen/ abgerufenen reparatur-relevanten Informationen abgeleitet. Die abgeleitete Arbeitsanweisung wird anschließend an einen Benutzer ausgegeben. Vorzugsweise wird überprüft, ob die Maßnahme abgeschlossen ist bzw. (korrekt) durchgeführt wurde. Die Aufgabe der vorliegenden Offenbarung wird ferner durch ein System mit dem medizinischen Gerät und der zentralen Datenverarbeitungseinheit gelöst. Das medizinische Gerät weist die geräte-spezifischen Informationen auf. Die zentrale Datenverarbeitungseinheit weist die reparaturrelevanten Informationen über das medizinische Gerät auf, die bevorzugt mit den geräte-spezifischen Informationen, beispielsweise in einer Datenbank, verknüpft sind. Das offenbarungsgemäße System ist eingerichtet, beispielsweise über eine Steuereinheit des medizinischen Geräts und/oder die zentrale Datenverarbeitungseinheit, das offenbarungsgemäße Verfahren auszuführen.

In anderen Worten wird durch das offenbarungsgemäße Verfahren ein gerätespezifischer und/oder varianten-spezifischer (Schritt-für-Schritt-) Plan zur Reparatur/ Instandsetzung/ Service/ Wartung eines medizinischen Geräts erzeugt. Dazu wird eine Information erfasst, die dazu geeignet ist, dem Gerät eine (Produkt-)Variante/ Version/ Modell zuordnen. Aufgrund der Information, die die Variante anzeigt, wird eine weitere Information abgerufen, aus der abzuleiten ist, wie die entsprechende Variante zu reparieren bzw. zu warten ist. Basierend auf dieser weiteren Information über die Reparatur bzw. Wartung der speziellen Variante des Geräts, wird eine (variantenspezifische) Arbeitsanweisung für genau diese Variante des Geräts bzw. der Baugruppe des Geräts abgleitet/ ausgelesen. Die Arbeitsanweisung wird für den Benutzer ausgegeben bzw. dem Benutzer als Handlungsanweisung angezeigt.

Vorzugsweise kann die Arbeitsanweisung von einer Steuerungseinheit des medizinischen Geräts und/oder von einer zentralen Datenverarbeitungseinheit abgeleitet werden. Die Arbeitsanweisung kann aber auch von einem separaten Gerät, beispielsweise einem Handgerät, wie einem Smartphone abgeleitet werden. In diesem Fall, kann die abgeleitete Arbeitsanweisung über den Bildschirm des Handgeräts ausgegeben werden. Alternativ kann die Arbeitsanweisung über den Bildschirm des medizinischen Geräts ausgegeben werden.

Bei der Arbeitsanweisung kann es sich beispielsweise um eine Anweisung/ Anleitung handeln, die dem Benutzer vorgibt, wie eine Maßnahme (an dem medizinischen Gerät) durchzuführen ist. Bei der Maßnahme kann es sich insbesondere um eine Reparatur/ Instandsetzung bzw. eine Wartung/ Instandhaltung des medizinischen Geräts handeln. Die Wartung/ Instandhaltung kann auch ein Aufspielen/ Installation eines Software-Updates oder eine Installation/ Montage einer Erweiterung des medizinischen Geräts beinhalten. Durch das Befolgen der abgeleiteten Arbeitsanweisung kann eine fehlerfreie, zügige und reibungslose Reparatur oder Wartung sichergestellt werden. Ferner kann durch das Befolgen der Arbeitsanweisung ein reproduzierbares Ergebnis der Maßnahme sichergestellt werden.

Geräte-spezifische Informationen können dabei insbesondere Informationen sein, durch die sich das medizinische Gerät eindeutig zuordnen lässt. Durch die geräte-spezifischen Informationen kann dabei insbesondere ein bestimmtes Gerät einer (Produkt)Variante/ einem Modell/ einer (Software-)Version/ einer Produktionslinie zugeordnet werden. Beispiele für die geräte-spezifischen Informationen können dabei insbesondere eine Seriennummer, eine Modellnummer und/oder ein Unique Identifier (UID) sein. Ferner können die geräte-spezifischen Informationen auch Informationen sein, die Aufschluss über die installierte Softwareversion bzw. ein Softwareupdate oder vorherige Reparaturen und/oder Instandhaltungen geben können. Die geräte-spezifischen Informationen können entweder auf dem medizinischen Gerät gespeichert sein und von dem medizinischen Gerät abgerufen werden oder können von dem Benutzer in eine Eingabeeinheit (des medizinischen Geräts) eingegeben werden.

Die reparatur-relevanten Informationen können dabei insbesondere Informationen sein, die angeben, wie eine bestimmte Variante repariert oder gewartet werden kann. Dabei können die reparatur-relevanten Informationen insbesondere angeben, was sich bei der bestimmten Variante im Vergleich zu einer anderen Variante geändert hat und wie sich diese Änderung auf die Reparatur/ Wartung auswirkt. Insbesondere können die reparatur-relevanten Informationen angeben, welche Abhängigkeiten oder Verknüpfungen zwischen einzelnen (neuen) Bauteilen/ Baugruppen bestehen.

Konkret kann eine solche Verknüpfung aussagen, dass ein bestimmtes Bauteil nur mit einem anderen Bauteil ab einer bestimmten Produktnummer kompatibel ist. D.h. wenn das eine Bauteil eingebaut wird, kann auch ein Austausch des anderen Bauteils notwendig sein. Diese Abhängigkeit muss in bekannten Verfahren händisch aus dem Handbuch abgeleitet werden. Die reparatur-relevanten Informationen können die Abhängigkeit zwischen den beiden Bauteilen in computerlesbarer Form abspeichern. Bei der Abhängigkeit kann es sich auch um eine Verknüpfung zwischen einem neu eingebauten Bauteil und einem Softwareupdate handeln, das für die Funktion des neuen Bauteils notwendig ist. In einem konkreten Beispiel kann ein neuer Bildschirm für eine Infusionspumpe nur mit einer neuen Version einer Gehäuseoberschale, die eine Tastatur aufweist, kompatibel sein. Der Zusammenhang zwischen der Kompatibilität (der Produktnummer) des Bildschirms und der benötigten Version der Gehäuseoberschale kann als die reparatur-relevanten Informationen abgespeichert und abrufbar sein. Die abgeleitete Arbeitsanweisung kann in diesem Fall dann beinhalten, dass bei einem Austausch des Bildschirms auch die Gehäuseoberschale ausgetauscht werden sollte.

Das medizinische Gerät kann dabei insbesondere eine medizinische Pumpe, wie beispielsweise eine Spritzenpumpe oder eine Infusionspumpe sein. Bei dem medizinischen Gerät kann es sich ferner um eine Dialysemaschine oder Dialysepumpe handeln. Auch andere Ausführungsformen des medizinischen Geräts sind grundsätzlich denkbar.

Die zentrale Datenverarbeitungseinheit kann dabei insbesondere ein Server bzw. ein Computer sein. Ferner kann die zentrale Datenverarbeitungseinheit auch eine Datenbank oder ein Datenspeicher sein, auf dem die geräte-spezifischen Informationen mit den reparatur-relevanten Informationen verknüpft sind. Durch die Verknüpfung der geräte-spezifischen Informationen mit den reparatur-relevanten Informationen können die Informationen, die für die Reparatur eines bestimmten Geräts relevant sind, für das jeweilige Gerät aus der zentralen Datenverarbeitungseinheit abgerufen werden.

Die Überprüfung, ob die Maßnahme abgeschlossen ist, kann dabei insbesondere durch das medizinische Gerät (automatisch) durchgeführt werden, etwa durch ein geeignetes Erfassen und/oder (Ein-)Scannen, ob die Maßnahme von einem Benutzer geeignet durchgeführt und abgeschlossen wurde. Es können beispielsweise eine geeignete Erfassungs-Sensorik und/oder geeignete Scannvorrichtungen in dem medizinischen Gerät verbaut sein. Ferner kann der Benutzer auf der Eingabeeinheit bestätigen, dass die Maßnahme durchgeführt wurde. Dazu kann der Benutzer einen Haken auf der Eingabeeinheit setzen oder einen Bestätigungskopf drücken. Der Benutzer kann das (erfolgreiche) Durchführen der Maßnahme auch mit Fotos und/oder einem Upload eines Wartungsprotokolls bestätigen.

Das Verfahren kann derart ausgestaltet sein, dass dem Benutzer auf dem Bildschirm des medizinischen Geräts oder des Handgeräts eine Schritt-für-Schritt Arbeits-/ Reparatur-/ Instandhaltungs-/ Wartungsanweisung ausgegeben bzw. angezeigt wird. Dies hat den Vorteil, dass der Benutzer Schritt-für-Schritt durch den Arbeits-/ Reparatur-/ Instandhaltungs-/ Wartungsprozess geleitet wird und vorzugsweise zu keinem Zeitpunkt mit einer unnötigen Vielzahl an Informationen überfordert wird.

Ferner kann vorgesehen sein, dass dem Benutzer die Arbeits-/ Reparatur-/ Instandhaltungs-/ Wartungsanweisung auf dem Bildschirm des medizinischen Geräts oder des Handgeräts durch bewegte oder unbeweglicher Bilder, etwa Fotos oder Videos, angezeigt bzw. veranschaulicht wird. Dies vereinfacht den Arbeits-/ Reparatur-/ Instandhaltungs-/ Wartungsprozess in großem Maße, da dem Benutzer der nächste durchzuführende Prozessschritt, unmittelbar bevor er diesen durchführen muss, auf dem Bildschirm angezeigt wird. Der Benutzer wird somit in vorteilhafter Weise Schritt-für-Schritt durch den Arbeits-/ Reparatur-/ Instandhaltungs-/ Wartungsprozess geführt.

In vorteilhafter Weise ist das Verfahren ferner derart ausgestaltet, dass der Arbeits-/ Reparatur-/ Instandhaltungs-/ Wartungsprozess, insbesondere die durchgeführten Schritte, automatisch dokumentiert werden, bevorzugt durch die zentrale Datenverarbeitungseinheit oder durch die Steuereinheit des medizinischen Geräts. Etwa erfolgt nach jedem durchgeführten Schritt/ nach jeder durchgeführten Maßnahme eine automatische Dokumentation des Schritts/ der Maßnahme durch die zentrale Datenverarbeitungseinheit oder durch die Steuereinheit des medizinischen Geräts. Somit wird in vorteilhafter Weise das Risiko einer fehlerhaften manuellen Dokumentation minimiert bzw. ausgeschlossen.

Das Verfahren stellt bevorzugt ein Anbinden von weiteren Systemen über Schnittstellen bereit. Etwa können über Schnittstellen ein Enterprise-Resource-Planning bzw. Unternehmens-Informations- und Prozesssystem und/oder ein digitales Gerätebuch und/oder ein Serviceportal an die zentrale Datenverarbeitungseinheit und/oder das medizinische Gerät angebunden sein. Beispielsweise können Daten über einen ursprünglichen Bauzustand des medizinischen Geräts oder einer Komponente bzw. eines Produkts des medizinischen Geräts durch das Enterprise-Resource-Planning bzw. Unternehmens-Informations- und Prozesssystem bereitgestellt werden. Bei diesen Daten kann es sich beispielsweise um einen Artikel, einen Herstellbericht, eine Chargeninformation, etc. handeln. Außerdem können Daten über einen nach einer Auslieferung erfolgten Reparaturfall des medizinischen Geräts oder einer Komponente bzw. eines Produkts des medizinischen Geräts durch das digitale Gerätebuch bereitgestellt werden. Ferner können Daten bezüglich Anweisungen und Konfigurationen aus einem Service Manual, welches beispielsweise über ein Serviceportal zur Verfügung gestellt wird, bereitgestellt werden. Das Verfahren stellt bevorzugt aus der Vielzahl von verfügbaren Daten, insbesondere unter Anwendung von Datenbanken, Algorithmen, KI, etc., eine eindeutige und spezifisch für das vorliegende Produkt/ das vorliegende medizinische Gerät gültige Schritt-für-Schritt (Reparatur-/ Arbeits-/ Instandhaltungs-/ Wartungs-) Anweisung bereit, das heißt leitet diese Anweisung ab und zeigt sie dem Benutzer auf einem Bildschirm an.

Der Kern der Offenbarung liegt also zusammenfassend darin, dass die Informationen, die für die Reparatur eines bestimmten medizinischen Geräts relevant sind, ausgelesen werden können und anhand dieser Informationen für das bestimmte medizinische Gerät eine (individualisierte) Reparaturanweisung/ Arbeitsanweisung ausgegeben wird, wobei ferner eine Überprüfung eines Abschlusses einer entsprechenden Reparatur-/ Wartungs-/ Instandhaltungs-/ Arbeitsmaßnahme erfolgt.

Durch das offenbarungsgemäße Verfahren kann die individualisierte Arbeitsanweisung einfach und schnell (automatisiert) angezeigt werden. Der Benutzer, beispielsweise ein Servicetechniker, kann dadurch die Arbeitsanweisung für die Maßnahme, insbesondere Reparatur und/oder Wartung einsehen, ohne die Verknüpfung zwischen vorliegender Variante des medizinischen Geräts und deren spezieller Arbeitsanweisung händisch aus dem Handbuch auslesen zu müssen. Dadurch hat der Benutzer immer die richtige Arbeitsanweisung für die zu reparierende Variante vorliegen. Dadurch kann eine potentielle Fehlerquelle bei der Maßnahme minimiert und ferner Arbeitszeit durch den Benutzer eingespart werden. Durch die eingesparte Arbeitszeit können Wartungskosten minimiert werden. Eine erfolgreiche Durchführung der Maßnahme kann, beispielsweise von dem medizinischen Gerät, erfasst werden.

Das offenbarungsgemäße Verfahren kann insbesondere dann durchgeführt werden, wenn das medizinische Gerät nicht mit einem Patienten verbunden ist. D.h. bevor die Maßnahme (Reparatur/Wartung) am Gerät durchgeführt wird, wird eine Verbindung zum Patienten getrennt. Das offenbarungsgemäße Verfahren ist somit kein Verfahren zur chirurgischen oder therapeutischen Behandlung des menschlichen oder tierischen Körpers und auch kein am menschlichen oder tierischen Körper vorgenommenes Diagnostizierverfahren, da es durchgeführt wird, wenn kein Patient an dem medizinischen Gerät angeschlossen ist bzw. von dem medizinischen Gerät therapiert bzw. behandelt wird.

Vorzugsweise kann das medizinische Gerät eine Ein- und/oder Ausgabeeinheit und eine Steuereinheit aufweisen und über eine Datenverbindung mit der zentralen Datenverarbeitungseinheit verbunden sein. Über die Datenverbindung können insbesondere die geräte-spezifischen Informationen an die zentrale Datenverarbeitungseinheit übermittelt werden. D.h. konkret, dass das medizinische Gerät die geräte-spezifischen Informationen bzw. die geräte-spezifischen Informationen über Bauteile des medizinischen Geräts (direkt) über die Datenverbindung an die zentrale Datenverarbeitungseinheit übermitteln kann, bevorzugt entweder automatisch, wenn das medizinische Gerät selbst eine Reparatur-/ Wartungs-/ Instandhaltungsmaßnahme für notwendig erachtet bzw. als erforderlich feststellt, oder auf Anfrage/ Anforderung durch den Benutzer. Basierend auf den übermittelten geräte-spezifischen Informationen können die reparatur-relevanten Informationen für das jeweilige Gerät aus der zentralen Datenverarbeitungseinheit ausgelesen werden. Insbesondere können die ausgelesenen reparatur-relevanten Informationen von der zentralen Datenverarbeitungseinheit weiterverarbeitet werden und die abgeleitete Arbeitsanweisung über die Datenverbindung (von der zentralen Datenverarbeitungseinheit) an das medizinische Gerät übermittelt werden. Über die Ein- und/oder Ausgabeeinheit können sowohl die geräte-spezifischen Informationen eingegeben bzw. erfasst werden, als auch die Arbeitsanweisung, bevorzugt die Schritt-für-Schritt-Arbeitsanweisungen, an den Benutzer ausgegeben werden.

Die geräte-spezifischen Informationen können entweder in dem medizinischen Gerät hinterlegt sein oder über die Ein- und/oder Ausgabeeinheit (von dem Benutzer) eingegeben werden. Die Arbeitsanweisung kann auch von dem medizinischen Gerät aus den reparaturrelevanten Informationen der zentralen Datenverarbeitungseinheit abgeleitet werden.

Für die Datenübermittlung zwischen dem medizinischen Gerät und der zentralen Datenverarbeitungseinheit kann das medizinischen Gerät an eine IT-Infrastruktur angebunden sein und insbesondere über WLAN oder LAN oder eine ähnliche Datenverbindung mit der zentralen Datenverarbeitungseinheit datenübertragend verbunden sein.

Vorzugsweise kann das System auch ein Handgerät aufweisen. Das Handgerät kann vorzugsweise ein Smartphone oder ein Tablet-Computer oder ein ähnlicher Handheld-Device mit einer Eingabe- und einer Ausgabeeinheit sein. Das Handgerät kann insbesondere die geräte-spezifischen Informationen des medizinischen Geräts (über eine Eingabeeinheit) erfassen. Dazu kann das Handgerät die geräte-spezifischen Informationen insbesondere als ein Barcode optisch auslesen. Das Handgerät kann ferner ein Foto einer gerätspezifischen Zahlenreihe in die geräte-spezifischen Informationen umwandeln. Alternativ kann das Handgerät eine elektromagnetische Strahlung des medizinischen Geräts, beispielsweise in Form eines RFID-Tags/ RFID-Chips auslesen, um die geräte-spezifischen Informationen zu erfassen.

Das Handgerät kann die erfassten geräte-spezifischen Informationen an die zentrale Datenverarbeitungseinheit übermitteln, beispielsweise durch LAN, WLAN oder Bluetooth oder eine ähnliche (kabelgebundene oder kabellose) Datenübertragung. Das Handgerät kann die Arbeitsanweisung insbesondere über einen Bildschirm an den Benutzer ausgeben.

Durch das offenbarungsgemäße System kann dem Benutzer (automatisiert) eine korrekte und zu dem zu reparierenden Gerät passende Arbeitsanweisung ausgegeben werden.

Nach einem optionalen Aspekt der vorliegenden Offenbarung können die geräte-spezifischen Informationen beispielsweise eine Seriennummer, ein Barcode und/oder ein RFID-Tag sein. Vorzugsweise können die geräte-spezifischen Informationen optisch und/oder elektronisch erfasst werden. Die geräte-spezifischen Informationen können dabei elektronisch auf dem medizinischen Gerät gespeichert und somit elektronisch abrufbar sein. Die geräte-spezifischen Informationen können auch anderweitig auf dem medizinischen Gerät angebracht sein, sodass sie vom Benutzer manuell oder mithilfe des Handgeräts ausgelesen werden können.

Vorzugsweise kann der Schritt Erfassen der geräte-spezifischen Informationen ein Scannen eines Barcodes (auf dem medizinischen Gerät) mit dem Handgerät und/oder ein elektronisches Auslesen eines RFID-Tags beinhalten. Durch das Erfassen der geräte-spezifischen Informationen kann dem medizinischen Gerät eine bestimmte Produktvariante zugeordnet werden. Die geräte-spezifischen Informationen können somit die Verknüpfung zu der Arbeitsanweisung für genau diese Variante/ dieses Modell darstellen.

Nach einem weiteren optionalen Aspekt der vorliegenden Offenbarung können die reparatur-relevanten Informationen die Informationen sein, die für die Reparatur und/oder die Instandsetzung einer bestimmten Produktvariante/ eines bestimmten Modells benötigt werden. Insbesondere können die reparatur-relevanten Informationen die Abhängigkeiten zwischen den einzelnen Bauteilen/ Baugruppen des medizinischen Geräts definieren. Durch die reparatur-relevanten Informationen können die geräte-spezifischen Informationen mit der Information verknüpft werden, wie genau die vorliegende Gerätevariante repariert oder gewartet werden kann.

Vorteilhafterweise kann der Schritt Ausgeben der abgeleiteten Arbeitsanweisung ein schrittweises Ausgeben der einzelnen (Reparatur-)Schritte der Maßnahme beinhalten. Somit kann der Benutzer Schritt für Schritt durch die Maßnahme geführt werden. Somit können Fehler bei der Durchführung der Maßnahme minimiert werden.

Vorteilhafterweise kann nach dem Schritt Ausgeben der abgeleiteten Arbeitsanweisung ein Ergebnis der Maßnahme (durch den Benutzer) eingegeben werden. Die Eingabe kann insbesondere durch Aufnehmen eines Fotos des Ergebnisses und/oder durch eine manuelle Eingabe/ Bestätigung am medizinischen Gerät oder Handgerät erfolgen. Somit kann der Erfolg der Maßnahme dokumentiert werden. Durch eine (systemseitige) Aufforderung nach der Maßnahme kann auch verhindert werden, dass die Dokumentation vergessen oder übersprungen wird.

Nach einem weiteren optionalen Aspekt der vorliegenden Offenbarung kann nach einzelnen Schritten der Arbeitsanweisung ein Ergebnis des jeweiligen Schritts der Maßnahme eingegeben werden. Somit können auch die einzelnen Schritte der Maßnahme dokumentiert werden. Eine Steuerung kann somit auswerten, ob die Maßnahme korrekt durchgeführt wurde.

Vorzugsweise kann das Ergebnis an die zentrale Datenverarbeitungseinheit übermittelt und in der zentralen Datenverarbeitungseinheit gespeichert werden. Dadurch können durchgeführte Maßnahmen bzw. der Erfolg von durchgeführten Maßnahmen zentral gespeichert und abrufbar sein. Durch die zentrale Speicherung der Informationen können durchgeführte Maßnahmen leicht nachgewiesen und überwacht werden.

Vorzugsweise kann es sich bei der zentralen Datenverarbeitungseinheit um eine Datenbank handeln, die die geräte-spezifischen Informationen mit den reparaturrelevanten Informationen und damit mit der Arbeitsanweisung verknüpft. Die Arbeitsanweisung kann aber auch durch die Anwendung von Algorithmen und/oder KI generiert werden. Somit ist eine einfache und automatische Generierung der Arbeitsanweisung möglich.

Die Aufgabe der vorliegenden Offenbarung wird ferner durch ein computerlesbares Speichermedium gelöst, das Befehle umfasst, die bei Ausführung durch einen Computer diesen veranlassen, die Schritte des Verfahrens nach einem der vorstehenden Aspekte auszuführen.

Ferner betrifft die vorliegende Offenbarung ein Computerprogramm, umfassend Befehle, die bei Ausführung durch einen Computer diesen veranlassen, die Schritte des Verfahrens nach einem der vorstehenden Aspekte auszuführen.

### Kurzbeschreibung der Figuren

Fig. 1 zeigt eine schematische Darstellung eines Systems gemäß einer Ausführungsform der vorliegenden Offenbarung;
Fig. 2 zeigt ein Ablaufdiagramm eines Verfahrens gemäß der vorliegenden Offenbarung; und
Fig. 3 zeigt eine schematische Darstellung eines Systems gemäß einer weiteren Ausführungsform der vorliegenden Offenbarung.

### Detaillierte Beschreibung der Figuren

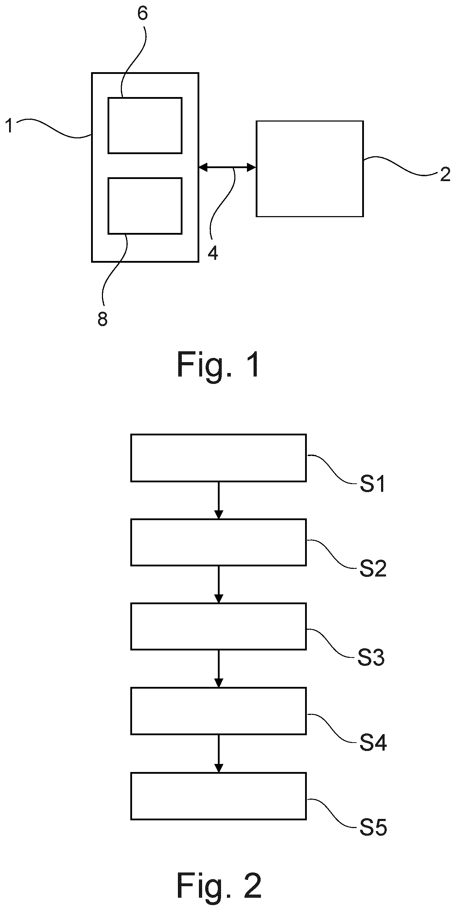

Fig. 1 zeigt ein System mit einem medizinischen Gerät 1 und einer zentralen Datenverarbeitungseinheit 2. Das medizinische Gerät 1 ist über eine Datenverbindung 4 mit der zentralen Datenverarbeitungseinheit 2 verbunden. Das medizinische Gerät 1 weist geräte-spezifischen Informationen auf. Die geräte-spezifischen Informationen werden über die Datenverbindung 4 zu der zentralen Datenverarbeitungseinheit 2 übermittelt. Basierend auf den geräte-spezifischen Informationen werden reparaturrelevanten Informationen aus der zentralen Datenverarbeitungseinheit 2 ausgelesen. Die ausgelesenen reparatur-relevanten Informationen werden entweder durch eine Steuereinheit 6 des medizinischen Geräts 1 oder durch die Datenverarbeitungseinheit 2 ausgewertet. Basierend auf den ausgelesenen reparatur-relevanten Informationen wird eine (gerätspezifische bzw. variantenspezifische) Arbeitsanweisung abgeleitet. Die abgeleitete Arbeitsanweisung wird über eine Ausgabeeinheit 8 des medizinischen Geräts 1 an den Benutzer ausgegeben. Bevorzugt werden abgeleitete Arbeitsanweisungen Schritt-für-Schritt über die Ausgabeeinheit 8 des medizinischen Geräts 1, bevorzugt über bewegte Bilder/ Videos, an den Benutzer ausgegeben.

Fig. 2 zeigt ein Ablaufdiagramm eines Verfahrens. Im Schritt S1 werden die geräte-spezifischen Informationen des medizinischen Geräts 1 erfasst und (automatisch oder manuell/ auf Anfrage bzw. Aufforderung durch einen Benutzer) von dem medizinischen Gerät 1 an die zentrale Datenverarbeitungseinheit 2 übermittelt. Basierend auf den übermittelten geräte-spezifischen Informationen werden im Schritt S2 reparatur-relevante Informationen aus der zentralen Datenverarbeitungseinheit 2 ausgelesen. Im Schritt S3 wird basierend auf den ausgelesenen reparatur-relevanten Informationen eine Arbeitsanweisung abgeleitet. Die abgeleitete Arbeitsanweisung ist dabei auf die übermittelten geräte-spezifischen Informationen angepasst/ abgestimmt. Somit ist die Arbeitsanweisung spezifisch für das medizinische Gerät 1. Im Schritt S4 wird die abgeleitete Arbeitsanweisung an einen Benutzer ausgegeben. Dabei kann die Arbeitsanweisung insbesondere als eine Schritt-für-Schritt Anweisung für die Reparatur und/oder Instandhaltung/ Wartung des medizinischen Geräts 1 sein. Im Schritt S5 wird überprüft, ob die Maßnahme/ die einzelnen Schritte (erfolgreich) durchgeführt wurde(n). Diese Überprüfung kann als Selbsttest von dem medizinischen Gerät 1 durchgeführt werden. Die Überprüfung kann aber auch durch den Benutzer durchgeführt werden. Der Benutzer kann die erfolgreiche Durchführung händisch bestätigen. Durch das Verfahren kann die (gerätspezifische bzw. variantenspezifische) Arbeitsanweisung schnell, einfach und automatisch generiert werden und an den Benutzer ausgegeben.

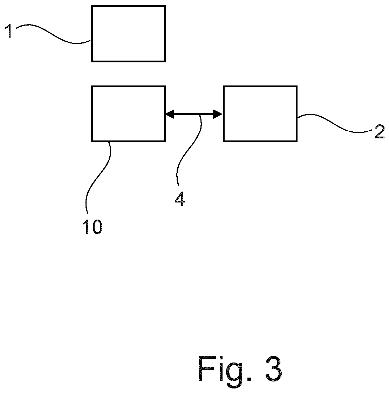

Fig. 3 zeigt eine schematische Darstellung eines Systems gemäß einer weiteren Ausführungsform. Das System weist das medizinische Gerät 1 und die zentrale Datenverarbeitungseinheit 2 auf. Das System weist ferner ein Handgerät 10, wie beispielsweise ein Smartphone oder ein Tablet, auf. Das Handgerät 10 erfasst die geräte-spezifischen Informationen des medizinischen Geräts 1 und übermittelt die erfassten geräte-spezifischen Informationen an die zentrale Datenverarbeitungseinheit 2. Die reparatur-relevanten Informationen werden entweder durch das Handgerät 10 oder durch die Datenverarbeitungseinheit 2 ausgewertet. Basierend auf den ausgelesenen reparatur-relevanten Informationen wird eine (gerätspezifische bzw. variantenspezifische) Arbeitsanweisung abgeleitet. Die abgeleitete Arbeitsanweisung wird über einen Bildschirm des Handgeräts 10 an den Benutzer ausgegeben.

## Patentansprüche

1. Verfahren zur Generierung und Ausgabe einer Arbeitsanweisung, insbesondere für eine Maßnahme an einem medizinischen Gerät (1), mit den folgenden Schritten:
- Erfassen von geräte-spezifischen Informationen eines medizinischen Geräts (1) und/oder eines Bauteils des medizinischen Geräts (1);
- Auslesen oder Abrufen von reparatur-relevanten Informationen basierend auf den erfassten geräte-spezifischen Informationen, insbesondere aus einer zentralen Datenverarbeitungseinheit (2);
- Ableiten der Arbeitsanweisung aus den ausgelesenen oder abgerufenen reparatur-relevanten Informationen; und
- Ausgeben der abgeleiteten Arbeitsanweisung an einen Benutzer, insbesondere über eine Ausgabeeinheit (8);
wobei das Verfahren vorzugsweise ferner den Schritt enthält:
- Überprüfen, insbesondere durch das medizinische Gerät (1), ob die Maßnahme abgeschlossen ist.

2. Verfahren nach Anspruch 1, **dadurch gekennzeichnet, dass** nach dem Schritt Ausgeben der abgeleiteten Arbeitsanweisung ein Ergebnis der Maßnahme eingegeben wird.

3. Verfahren nach Anspruch 2, **dadurch gekennzeichnet, dass** das Ergebnis der Maßnahme an die zentrale Datenverarbeitungseinheit (2) übermittelt und in der zentralen Datenverarbeitungseinheit (2) gespeichert wird.

4. Verfahren nach einem der Ansprüche 1 bis 3, **dadurch gekennzeichnet, dass** die geräte-spezifischen Informationen eine Seriennummer, ein Barcode und/oder ein RFID-Tag sind, wobei die geräte-spezifischen Informationen insbesondere in dem medizinischen Gerät (1) elektronisch hinterlegt sind.

5. Verfahren nach einem der Ansprüche 1 bis 4, **dadurch gekennzeichnet, dass** der Schritt Erfassen der geräte-spezifischen Informationen ein optisches oder elektronisches Erfassen aufweist, insbesondere ein Scannen eines Barcodes mit einem Handgerät (10), insbesondere einem Smartphone, und/oder ein elektronisches Auslesen eines RFID-Tags mit dem Handgerät (10).

6. Verfahren nach einem der Ansprüche 1 bis 5, **dadurch gekennzeichnet, dass** die reparatur-relevanten Informationen solche Informationen sind, die für eine Maßnahme an dem medizinischen Gerät (1), insbesondere eine Reparatur und/oder eine Instandhaltung des medizinischen Geräts (1) benötigt werden, und insbesondere Abhängigkeiten zwischen den einzelnen Bauteilen des medizinischen Geräts beinhalten.

7. Verfahren nach einem der Ansprüche 1 bis 6, **dadurch gekennzeichnet, dass** der Schritt Ausgeben der abgeleiteten Arbeitsanweisung ein schrittweises Ausgeben beinhaltet.

8. Verfahren nach Anspruch 7, **dadurch gekennzeichnet, dass** jeweils nach den einzelnen Schritten der Arbeitsanweisung das Ergebnis des jeweiligen Schrittes der Maßnahme eingegeben wird.

9. System mit einem medizinischen Gerät (1) aufweisend geräte-spezifische Informationen und einer zentralen Datenverarbeitungseinheit (2), die reparatur-relevante Informationen über das medizinische Gerät (1) aufweist, wobei das System zur Ausführung des Verfahrens nach einem der vorhergehenden Ansprüche 1 bis 8 eingerichtet ist.

10. System nach Anspruch 9, **dadurch gekennzeichnet, dass** das medizinische Gerät (1) geräte-spezifische Informationen, eine Ein- und/oder Ausgabeeinheit, eine Steuereinheit und eine Datenverbindung (4) aufweist und über die Datenverbindung (4) mit der zentralen Datenverarbeitungseinheit (2) verbunden ist und die geräte-spezifische Informationen über die Datenverbindung (4) an die zentrale Datenverarbeitungseinheit (2) übermittelt und eine Arbeitsanweisung über die Ein- und/oder Ausgabeeinheit ausgibt.

11. System nach Anspruch 9 oder 10, **gekennzeichnet durch** ein Handgerät (10), vorzugsweise ein Smartphone, das die geräte-spezifischen Informationen des medizinischen Geräts (1) erfasst, die erfassten geräte-spezifischen Informationen an die zentrale Datenverarbeitungseinheit (2) übermittelt, und eine Arbeitsanweisung an einen Benutzer ausgibt, insbesondere über einen Bildschirm des Handgeräts (10).

12. Computerlesbares Speichermedium, umfassend Befehle, die bei Ausführung durch einen Computer diesen veranlassen, die Schritte des Verfahrens nach einem der Ansprüche 1 bis 8 auszuführen.

13. Computerprogramm, umfassend Befehle, die bei Ausführung durch einen Computer diesen veranlassen, die Schritte des Verfahrens nach einem der Ansprüche 1 bis 8 auszuführen.
